# EUROPÄISCHE PATENTSCHRIFT

(11) **EP 3 060 543 B1**
(45) Veröffentlichungstag und Bekanntmachung des Hinweises auf die Patenterteilung: **25.07.2018**
(21) Anmeldenummer: 14786934.1
(22) Anmeldetag: 22.10.2014
(51) Int. Cl.: C07C 201/16, C07C 205/06

(54) **VERFAHREN ZUR SAUREN WÄSCHE VON DINITROTOLUOL IN GEGENWART VON BLAUSÄURE**
METHOD FOR THE ACIDIC WASHING OF DINITROTOLUENE IN THE PRESENCE OF HYDROGEN CYANIDE
PROCÉDÉ DE LAVAGE ACIDE DE DINITROTOLUÈNE EN PRÉSENCE D'ACIDE CYANHYDRIQUE

(30) Priorität: 22.10.2013 EP 13189687
(43) Veröffentlichungstag der Anmeldung: 31.08.2016
(73) Patentinhaber: BASF SE, 67056 Ludwigshafen am Rhein (DE); Josef Meissner GmbH & Co. KG, 50968 Köln (DE)
(72) Erfinder: NETO, Samuel, Manila Alabang City 1781 (PH); FRITZ, Rüdiger, 02994 Bernsdorf Strassgräbchen (DE); HEMPEL, Renate, 01945 Ruhland (DE); ALLARDT, Holger, 01987 Schwarzheide (DE); DAI, Yuanshen, 68165 Mannheim (DE); BECKER, Barbara, 69509 Mörlenbach (DE); AHRENS, Sebastian, 69168 Wiesloch (DE); LESCHINSKI, Julia, B-1050 Ixelles (Bruxelles) (BE); HERMANN, Heinrich, 50858 Köln (DE); HÄNDEL, Mirko, 53819 Neunkirchen-Seelscheid (DE); PÖHLMANN, Jürgen, 50969 Köln (DE)
(74) Vertreter: Kudla, Karsten
(86) Internationale Anmeldenummer: PCT/EP2014/072633
(87) Internationale Veröffentlichungsnummer: WO 2015/059185

(56) Entgegenhaltungen:
- EP-A1- 0 736 514
- EP-A1- 1 780 195
- WO-A1-2013/160367
- US-A- 4 361 712

## Beschreibung

Die Erfindung betrifft ein Verfahren zur Wäsche eines bei der Nitrierung von Toluol nach Abtrennung der Nitriersäure anfallenden Rohgemischs enthaltend Dinitrotoluol (DNT), Salpetersäure, Stickstoffoxide und Schwefelsäure.

Die vorliegende Erfindung betrifft insbesondere ein Verfahren zur Wäsche des DNT enthaltenden Rohgemischs (nachfolgend auch als Roh-DNT bezeichnet) aus einer kontinuierlichen isothermen Nitrierung von Toluol zu DNT in einem Gemisch von Schwefel- und Salpetersäure, das es gestattet, das resultierende Waschwasser aus dieser Wäsche nach Entfernung von in diesem Wasser gelöstem DNT und Blausäure, ohne weitere physikalische oder chemische Vorbehandlung, einer biologischen Behandlung in einer Kläranlage zu unterwerfen, wobei das aus der Kläranlage ablaufende Abwasser die Anforderungen bezüglich Toxizität, wie sie z.B. in der Abwasserverordnung der Bundesrepublik Deutschland, Anhang 22 : Chemische Industrie, vorgegeben sind, erfüllt.

Bei der kontinuierlichen isothermen oder adiabatischen Nitrierung von Toluol zu DNT in einer oder zwei Stufen im Gegenstrom mit Mischsäure wird nach Phasentrennung stets ein Rohprodukt an Nitroaromat erhalten, das vor Weiterverwendung von den darin gelösten Verunreinigungen befreit werden muss. Neben der im Nitroaromat gelösten bzw. als Mikroemulsion vorliegenden Nitrierendsäure aus Salpetersäure, Schwefelsäure und Stickoxiden sind noch Oxidationsprodukte aus Nebenreaktionen mit dem zu nitrierenden Aromaten, wie Mono-, Di- und Trinitrokresole, oder aromatischen Carbonsäuren, wie z.B. Mono- und Dinitrobenzoesäuren (im Folgenden als Nitrobenzoesäuren oder NBS bezeichnet) und deren Abbauprodukte, im Roh-DNT enthalten.

Bei der Nitrierung von Toluol zu Nitrotoluolen werden neben den gewünschten Nitrokörpern als Nebenprodukte u. a. gebildet: Mono- (MNK), Di- (DNK) und Trinitrokresole (TNK), Trinitrophenol (Pikrinsäure PS), Nitrobenzoesäuren, wie Mononitrobenzoesäure (MNBS) und Dinitrobenzoesäure (DNBS), oxidative Abbauprodukte der im Toluol vorhandenen Spuren an aliphatischen/cycloaliphatischen Kohlenwasserstoffe, der Nitrokresole und Nitroaromaten, wie Kohlendioxid (CO₂), Kohlenmonoxid (CO), Blausäure (HCN), Tetranitromethan (TNM), Ameisensäure, Essigsäure und Oxalsäure, und Reduktionsprodukte der Salpetersäure wie Stickoxide (NO₂/NO) und Distickstoffoxid (N₂O), die alle in dem Nitrotoluol (MNT, DNT) nach Abtrennung der Nitrierendsäure gelöst sein können.

Die Bildung all dieser Neben- und Abbauprodukte erfolgt nicht auf allen Nitrierstufen in gleichem Ausmaß, sondern hängt, wie die bevorzugte Bildung der einzelnen Nitroaromaten auch, von dem Wassergehalt in der Mischsäure ab. So werden z.B. auf der MNT-Stufe mit Mischsäuren mit hohem Gehalt an Wasser bevorzugt die Mono- und Dinitrokresole und weniger Oxidationsprodukte, wie Nitrobenzoesäure, gebildet. Auf der DNT-Stufe werden, neben der Neubildung von Nitrokresolen, bevorzugt die Mono- und Dinitrokresole aus der MNT-Stufe und die im Toluol enthaltenen aliphatischen Kohlenwasserstoffe mit Salpetersäure oxidativ abgebaut, wobei neben CO, CO₂ und nicht vollständig oxidierten Abbauprodukten wie Oxalsäure, Essigsäure, Ameisensäure, den Stickoxiden NOx und N₂O auch in Spuren Blausäure gebildet wird.

So wird in US 4 361 712 gezeigt, dass auf der DNT-Stufe, neben anderen Abbauprodukten wie z.B. CO₂, CO und niedrigmolekularen Carbonsäuren, in geringen Mengen auch das starke Umwelt- und Katalysatorgift Blausäure durch Oxidation der auf der MNT-Stufe gebildeten Mono-und Dinitrokresole gebildet wird. Aus einem MNT mit einem Gehalt von 0,3 bis 0,6 Gew.-% an Mono- und Dinitrokresolen wird, nach Wäsche des DNT mit Wasser entsprechend einer sauren Wäsche (Waschschritt I), ein Waschwasser erhalten, das 86 ppm an HCN enthält. Über den Restgehalt an Blausäure im mit Wasser gewaschenen DNT werden keine Angaben gemacht.

DNT wird üblicherweise, gemäß dem Stand der Technik, ausgehend von Toluol mit Salpetersäure in Gegenwart von Schwefelsäure (Mischsäure) isotherm in zwei Stufen im Gegenstrom zu DNT umgesetzt. Aber auch eine adiabatische Reaktionsführung in einer oder zwei Stufen ist beschrieben worden. Eine Abtrennung der bei zweistufiger Nitrierung auf der MNT-Stufe gebildeten Nitrokresole erfolgt üblicherweise nicht, so dass bei den Verfahren nach dem Stand der Technik obligatorisch die Nitrokresole in der DNT-Stufe zum größten Teil oxidativ abgebaut werden, wobei in geringem Ausmaß auch Blausäure gebildet wird. Im Roh-DNT, nach dem Stand der Technik hergestellt, ist daher stets Blausäure in Spuren von 30 bis 120 ppm vorhanden. Die aktuelle Menge hängt dabei ab von der Menge der in der MNT-Stufe gebildeten Nitrokresole und den Nitrierbedingungen in der DNT-Stufe.

Viele dieser Neben- und Abbauprodukte, die im Verlaufe der Nitrierung von Toluol zu MNT oder DNT gebildet werden, sind hochtoxisch und Katalysatorgifte in der Hydrierung der Nitrotoluole zu den entsprechenden Aminen. Sie müssen deshalb vor einer katalytischen Hydrierung aus dem Nitrotoluol entfernt werden. Üblicherweise wird das rohe Nitrotoluol, nach Abtrennung der Nitrierendsäure, in drei Waschschritten gewaschen, wobei ein Waschschritt in Gegenwart von Alkali bei einem pH von 8 bis 12 durchgeführt wird. Diese Waschschritte nach dem Stand der Technik sind:
1. eine saure Wäsche zur Entfernung der gelösten und suspendierten Mineralsäuren wie Schwefelsäure, Salpetersäure und von Stickstoffoxiden (saure Wäsche);
2. eine Alkaliwäsche in Gegenwart einer Base (Alkaliwäsche) wie Natriumcarbonat (Soda), Natriumbicarbonat, Ammoniak , Natronlauge oder Kalilauge (siehe US 4 482 769, US 4 597 875, US 6 288 289) zur Entfernung der im rohen Nitroaromaten gelösten schwach aciden Verunreinigungen wie der Nitrokresole, Nitrobenzoesäuren und Abbauprodukte aus dem oxidativen Abbau der Nitrokresole oder von aliphatischen oder cyclischen Kohlenwasserstoffen, wie z.B. Oxalsäure, Blausäure, CO₂;
3. eine Neutralwäsche zur Entfernung der Alkali-Restspuren und der weiteren Reduzierung der noch in Spuren im Produkt verbliebenen Verunreinigungen. Als Waschmedium wird dafür üblicherweise Wasser eingesetzt, und die Wäsche wird als Flüssig/flüssig-Wäsche bei den Temperaturen, bei denen der zu waschende Nitroaromat als Flüssigkeit vorliegt, durchgeführt.

EP 1 780 195 A1 offenbart ein Verfahren zum Entfernen und Wiedergewinnen von Nitriersäuregemischen aus dem bei der Nitrierung von nitrierbaren aromatischen Verbindungen nach Abtrennung der Nitriersäure anfallenden nitrierten Rohprodukten durch ein mehrstufiges Extraktionsverfahren. Das Extraktionsverfahren umfasst eine Querstromextraktion und eine im Anschluss daran durchgeführte Gegenstromextraktion. Das aus der Querstromextraktion stammende wässrige Säuregemisch aus Schwefelsäure, Salpetersäure und salpetriger Säure mit einem Gesamtsäuregehalt von 40 bis 80 Gew.-% wird direkt oder nach Aufkonzentrierung der Nitrierung wieder zugeführt. Das aus der Gegenstromextraktion abgezogene Säuregemisch aus Schwefelsäure, Salpetersäure und salpetriger Säure weist einen Gesamtsäuregehalt, berechnet als Salpetersäure, von 15 bis 40 Gew.-% auf. Die Gegenstromextraktion wird mehrstufig, vorzugsweise 2- bis 4-stufig durchgeführt.

EP 0 736 514 A1 offenbart ein Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen, bei dem die rohen Dinitrotoluole mit einer verdünnten wässrigen Lösung von Salpetersäure, Schwefelsäure und salpetriger Säure mehrstufig im Gegenstrom extrahiert werden, und das wässrige Extrakt direkt oder nach Aufkonzentrierung in die Nitrierung zurückgeführt wird.

Aufgabe der Erfindung ist es, ein Verfahren zur Wäsche des bei der Nitrierung von Toluol nach Abtrennung der Nitriersäure anfallenden, Dinitrotoluol enthaltenden Rohgemischs bereitzustellen, das es gestattet, das Abwasser aus dieser Wäsche direkt und ohne aufwendige Zwischenbehandlung zur Abtrennung bzw. Zerstörung der Nitrokresole und Nitrobenzoesäuren in die biologische Behandlungsstufe einer Kläranlage abzugeben. Das in der biologischen Stufe behandelte Abwasser soll, unter Einhaltung aller einschlägigen gesetzlichen Auflagen, z.B. der Vorgaben der Abwasserabgabeverordnung der Bundesrepublik Deutschland, in einen Vorfluter eingeleitet werden können. Insbesondere ist es Aufgabe der Erfindung, ein Verfahren zur Wäsche des DNT-haltigen Rohgemischs bereitzustellen, bei dem die in dem DNT-haltigen Rohgemisch vorliegenden Nitrobenzoesäuren (Mono- und Dinitrobenzoesäuren) nicht in das Abwasser der Wäsche gelangen. Weiterhin ist es Aufgabe der Erfindung, ein solches Verfahren bereitzustellen, bei dem nach der Wäsche ein "technical grade DNT" erhalten wird, in dem die aus dem oxidativen Abbau der Nitrokresole auf der DNT-Stufe der Nitrierung anfallenden, bekannten Abbauprodukte und potentiellen Katalysatorgriffe, die bei der katalytischen Reduktion des DNT mit Wasserstoff stören können, wie Distickstoffoxid (N₂O), Stickoxide (NOx) und Kohlenmonoxid (CO), minimiert werden. Weiterhin ist es die Aufgabe der Erfindung, die im Roh-DNT gelöste Blausäure soweit aus dem DNT im Verlauf der Wäsche zu entfernen, dass bei der Reduktion des DNT zu Toluylendiamin (TDA) in Gegenwart eines Katalysators keine Beeinträchtigungen durch die im gewaschenen DNT noch vorhandene Blausäure beobachtbar sind. Weiterhin besteht die Aufgabe der Erfindung darin, ein Verfahren bereitzustellen, bei dem der Gehalt an Schwefelsäure, Salpetersäure und Nitrose (als HNO₂) im in die Kläranlage abzugebendem Abwasser minimiert werden und der Gehalt an Blausäure so weit reduziert wird, dass ein störungsfreier Betrieb der Kläranlage mit diesem Abwasser stets gewährleistet ist.

Gelöst wird die Aufgabe durch ein Verfahren zur Wäsche eines bei der Nitrierung von Toluol nach Abtrennung der Nitriersäure anfallenden Rohgemischs enthaltend Dinitrotoluol, Salpetersäure, Stickstoffoxide und Schwefelsäure umfassend zwei Waschschritte (WS-I) und (WS-II), wobei
i) in einem ersten Waschschritt (WS-I) das Rohgemisch in einer mindestens eine Extraktionsstufe umfassenden Wäsche mit einer Salpetersäure, Stickstoffoxide und Schwefelsäure enthaltenden Waschsäure I extrahiert wird, wobei die aus der ersten Extraktionsstufe (WS-I-1) des ersten Waschschrittes (WS-I) abgeführte Waschsäure einen Gesamtsäuregehalt von 10 bis 40 Gew.-% und einen Gehalt an 80 bis 350 ppm Blausäure aufweist, wobei ein vorgewaschenes Dinitrotoluol enthaltendes Rohgemisch erhalten wird,
ii) in einem zweiten Waschschritt (WS-II) das vorgewaschene Rohgemisch in einer mindestens eine Extraktionsstufe umfassenden Wäsche mit einer Waschsäure II extrahiert wird, wobei die aus der ersten Extraktionsstufe (WS-II-1) des zweiten Waschschrittes (WS-II) abgeführte Waschsäure einen pH-Wert von kleiner oder gleich 4 und einen Gehalt an Sulfat von maximal 100 ppm, einen Gehalt an Salpetersäure von maximal 2000 ppm, einen Gehalt an Stickstoffoxiden von maximal 50 ppm und einen Gehalt an Blausäure von maximal 120 ppm aufweist, wobei ein von Salpetersäure, Schwefelsäure, Stickstoffoxiden und Blausäure im wesentlichen freies Dinitrotoluol enthaltendes Gemisch mit einem Gehalt von maximal 300 ppm Salpetersäure und Stickstoffoxiden, maximal 3 ppm Sulfat und maximal 50 ppm Blausäure erhalten wird.

Überraschender Weise wurde gefunden, dass bei der erfindungsgemäßen sauren Wäsche umfassend einen ersten Waschschritt (WS-I) und einen zweiten Waschschritt (WS-II) und optional eine Extraktionsstufe mit Strippung
1) die Blausäure mit schwachen bis mittelstarken Säuren als Extraktionsmittel aus dem Roh-DNT derart ausgewaschen werden kann, dass die Nitrokresole, Nitrobenzoesäuren und die anderen organischen Verunreinigungen aus dem oxidativen Abbau der Nitrokresole im Roh-DNT verbleiben und ein DNT erhalten wird, aus dem die ursprünglich vorhandene Blausäure fast vollständig entfernt wurde, während die Nitrokresole und Nitrobenzoesäuren noch vollständig im DNT enthalten sind;
2) aus dem zweiten Waschschritt ein Abwasser erhalten wird, in dem nur so geringe Mengen Nitrokresolen (Dinitro- und Trinitrokresolen) und der Nitrobenzoesäuren (Mono- und Dinitrobenzoesäure) sowie von oxidativen Abbauprodukte der Dinitrokresole vorliegen, dass eine aufwendige Zwischenbehandlung nach dem Stand der Technik zur Entfernung derselben nicht mehr benötigt wird;
3) dass, nach Extraktion des Abwassers mit Toluol zur Rückgewinnung des in diesem Abwasser noch gelösten und suspendierten DNT, durch Strippung des extrahierten Abwassers zur Rückgewinnung des darin gelösten und suspendierten Toluols auch das in diesem Abwasser gelöste Cyanid (Blausäure) entfernt werden kann.

Weiterhin wurde gefunden, dass bei der erfindungsgemäßen sauren Wäsche umfassend einen ersten Waschschritt (WS-I) und einen zweiten Waschschritt (WS-II) in dem zweiten Waschschritt ein Abwasser erhalten wird, das ohne Zwischenbehandlung zur Abtrennung von Nitrokresolen (Dinitro- und Trinitrokresolen) und der Nitrobenzoesäuren (Mono- und Dinitrobenzoesäure) sowie von oxidativen Abbauprodukte der Dinitrokresole in die biologische Behandlungsstufe einer Kläranlage eingebracht werden kann. Das nach Verlassen der biologischen Behandlungsstufe erhaltene Abwasser kann direkt und unter Einhaltung der in einschlägigen Abwasserverordnungen, beispielsweise der Bundesrepublik Deutschland, vorgegebenen Grenzwerte in einen Vorfluter abgegeben werden. Insbesondere war überraschend, dass bei Einhaltung des pH-Wertes in der ersten Extraktionsstufe des zweiten Waschschritts sich nahezu die Gesamtmenge der Nitrobenzoesäuren in der DNT enthaltenden organischen Phase wiederfindet und nicht in das Abwasser übergeht.

In einem ersten Waschschritt (WS-I) wird das Rohgemisch in einer mindestens eine Extraktionsstufe umfassenden Wäsche mit einer Salpetersäure, Stickstoffoxide und Schwefelsäure enthaltenden Waschsäure (WSR-I) extrahiert wobei die aus der ersten Extraktionsstufe (WS-I-1) des ersten Waschschrittes (WS-I) abgeführte Waschsäure (WSR-I-1) einen Gesamtsäuregehalt von 10 bis 40 Gew.-% aufweist und einen Gehalt von 80-350 ppm an Blausäure (in Abhängigkeit vom Verhältnis Frischwasser/Roh-DNT), wobei ein vorgewaschenes Dinitrotoluol enthaltendes Rohgemisch erhalten wird.

In einem zweiten Waschschritt (WS-II) wird das vorgewaschene Rohgemisch in einer mindestens zwei Extraktionsstufen umfassenden Wäsche mit einer Waschsäure (WSR-II) extrahiert, wobei die aus der ersten Extraktionsstufe (WS-II-1) des zweiten Waschschrittes (WS-II) abgeführte Waschsäure (WSR-II-1) einen pH-Wert von kleiner oder gleich 4 aufweist, wobei ein von Salpetersäure, Schwefelsäure, Stickoxiden, Blausäure CO₂, CO und N₂O im wesentlichen freies Dinitrotoluol enthaltendes Gemisch, in dem noch die ursprünglich im Roh-DNT vorhandenen Nitrokresole, Nitrophenole und Nitrobenzoesäuren vorliegen, erhalten wird.

In der Extraktions-/Strippstufe wird die aus Waschschritt (WS-II) anfallende Waschsäure (WSR-II-1) mit Toluol extrahiert, um das gelöste und suspendierte DNT zurückzugewinnen. Anschließend wird, nach Phasentrennung, das noch gelöstes und suspendiertes Toluol enthaltende Wasser mit einem DNT-Gehalt von weniger als 20 ppm, bevorzugt weniger als 10 ppm und besonders bevorzugt weniger als 1 ppm, mit Dampf oder einem Inertgas gestrippt. Das in dem sauren Abwasser mit einem pH ≤ 4 vorliegende Cyanid wird dabei zusammen mit dem Toluol abgetrennt und im Strippkondensat gesammelt.

Durch diese Aufteilung der sauren Wäsche in zwei getrennte Waschschritte, in Verbindung mit einer Extraktion und Strippung, wird im Vergleich zum Stand der Technik, wie er beispielsweise in US 2 976 320, US 4 482 769 und CA 1 034 603 beschrieben ist, die Abwasserbelastung mit Sulfat und vor allem mit Nitrat, das nur aufwendig aus dem Abwasser entfernt werden kann, wesentlich reduziert.

Das erfindungsgemäße Verfahren stellt im Sinne der EU-Richtlinie zur integrierten Verminderung und Vermeidung von Umweltverschmutzung (IVU-RL) eine Weiterentwicklung der besten verfügbaren Techniken (BVT), wie sie in den BREFs (BVT-Referenz-Dokument) konkretisiert werden, dar. Mit dem erfindungsgemäßen Verfahren kann nicht nur die gesamte Abwassermenge aus einer DNT-Anlage deutlich reduziert werden, sondern es können auch die umfangreichen und technisch aufwendigen Maßnahmen zur Entfernung oder teilweisen Entfernung der Nitrokresole, Nitrobenzoesäuren und sonstigen Abbauprodukte aus dem Abwasser entfallen.

Dabei werden im ersten Waschschritt (WS-I) in einer mindestens einstufigen Extraktion mit einer Waschsäure als Extraktionsmittel die in dem DNT enthaltenden Rohgemisch gelösten Mineralsäuren Schwefelsäure und Salpetersäure sowie Stickstoffoxide abgetrennt.

Vorzugsweise wird der erste Waschschritt mindestens zweistufig und im Gegenstrom durchgeführt. Die mindestens zweistufige Gegenstromextraktion kann prinzipiell wie in EP 0 279 312, EP 0 736 514 oder EP 1 780 195 beschrieben erfolgen.

Im Gegenstrom wird der erste Waschschritt im Allgemeinen dergestalt durchgeführt, dass
I) in der ersten Extraktionsstufe (WS-I-1) des ersten Waschschritts (WS-I) das Rohgemisch enthaltend Dinitrotoluol, Salpetersäure, Stickstoffoxide und Schwefelsäure in einen ersten Mischer (M-I-1) zusammen mit im Kreis geführter Waschsäure (WSR-I-1) aus einem zu dieser ersten Extraktionsstufe gehörenden ersten Phasentrennapparat (S-I-1) und überschüssiger Waschsäure (WSR-I-2) aus der nachfolgenden Extraktionsstufe (WS-I-2) eingespeist wird, die in dem Mischer gebildete Waschemulsion in dem ersten Phasentrennapparat (S-I-1) in die Waschsäure (WSR-I-1) und einmal gewaschenes Rohgemisch (DNT-I-1) aufgetrennt, und
II) das einmal gewaschene Rohgemisch (DNT-I-1) in der nachfolgenden Extraktionsstufe (WS-I-2) des ersten Waschschritts (WS-I) in einen zu dieser Extraktionsstufe gehörenden zweiten Mischer (M-I-2) zusammen mit der im Kreis geführten Waschsäure (WSR-I-2) aus einem zu dieser Extraktionsstufe gehörenden zweiten Phasentrennapparat (S-I-2) und, wenn der erste Waschschritt (WS-I) mehr als 2 Extraktionsstufen umfasst, mit überschüssiger Waschsäure (WSR-I-3) aus der nachfolgenden Extraktionsstufe (WS-I-3) oder, wenn der erste Waschschritt genau 2 Extraktionsstufen umfasst, mit Frischwasser oder Waschsäure eingespeist wird, wobei, wenn der erste Waschschritt mehr als 2 Extraktionsstufen umfasst, sich weitere Extraktionsschritte entsprechend den Schritten I) und II) anschließen können.

Die Gegenstromextraktion kann bis zu n Extraktionsstufen aufweisen. Dabei wird das Extraktionsmittel der letzten der n Extraktionsstufen (WS-I-n) des ersten Waschschritts (WS-I) zugeführt. Das zugeführte Extraktionsmittel, das dieser letzten Extraktionsstufe zugeführt wird, kann Frischwasser oder eine Waschsäure mit einem bestimmten Gesamtsäuregehalt sein. In einer Ausführungsform der Erfindung ist das zugeführte Extraktionsmittel Wasser. In einer weiteren Ausführungsform ist das zugeführte Extraktionsmittel eine Waschsäure mit einem Gesamtsäuregehalt im Bereich von 0,2 bis 1,5 Gew.-%. Beispielsweise kann diese Waschsäure das Brüdenkondensat aus der Aufkonzentrierung der Waschsäure, die im ersten Extraktionsschritt (WS-I-1) der ersten Waschstufe (WS-I) gewonnen wird, sein. Diese enthält im Allgemeinen bis zu 1,0 Gew.-% Salpetersäure und bis zu 0,3 Gew.-% Schwefelsäure und bis zu 150 ppm an Blausäure, die aus der Waschsäure (WS-I-1) stammen. Aber auch das Brüdenkondensat aus der Anlage zur Wiederaufkonzentrierung der Schwefelsäure aus der Nitrierung (SAC-Anlage) mit bis zu 2 Gew.-% Gesamtsäure (vorwiegend Schwefelsäure) kann als Waschsäure eingesetzt werden.

Die in dem ersten Waschschritt in der ersten Extraktionsstufe (WS-I-1) anfallende Waschsäure weist im Allgemeinen einen Gesamtsäuregehalt von 10 bis 40 Gew.-% auf. Dieser Gesamtsäuregehalt ist die Summe aus Salpetersäure, Schwefelsäure und Stickoxide, wobei Stickoxide als Salpetrige Säure HNO₂ berechnet werden.

Überraschenderweise sind in dieser Waschsäure mit einem Gehalt von 10 bis 40%, bevorzugt 20-30 Gew. %, an Mineralsäure zusätzlich 80 bis 350 ppm an Blausäure gelöst, die aus dem Roh-DNT (30 bis 120 ppm Blausäure) ausgewaschen und mit der Waschsäure aus (WS-I-1) ausgetragen werden.

Die in dem ersten Waschschritt auf der ersten Extraktionsstufe anfallende Waschsäure kann direkt oder nach Aufkonzentrierung in die Nitrierung von Toluol zurückgeführt werden. Sie kann auch in die Aufkonzentrierung der Endsäure aus der ersten Stufe der Toluol-Nitrierung (Mononitrotoluol-Stufe) eingebracht werden. In diesem ersten Waschschritt (WS-I) wird im Wesentlichen die Belastung des im zweiten Waschschritt anfallenden Abwassers mit Sulfat und Nitrat minimiert. Letzteres kann nur durch eine aufwendige Denitrifizierungsstufe aus dem Abwasser entfernt werden.

Das nach dem ersten Waschschritt anfallende vorgewaschene, DNT enthaltende Rohgemisch weist im Allgemeinen einen Gehalt an Sulfat von maximal 300 ppm, bevorzugt von maximal 100 ppm und besonders bevorzugt von maximal 50 ppm und einem Gehalt an Salpetersäure und Stickoxiden von maximal 5000 ppm, bevorzugt von maximal 3000 ppm und besonders bevorzugt von maximal 1000 ppm auf. Der Restgehalt an Blausäure im vorgewaschenen DNT beträgt im Allgemeinen 20 bis 90 ppm, bevorzugt 30 bis 70 ppm.

Im zweiten Waschschritt (WS II) werden in einer mindestens einstufigen Extraktion mit einer Waschsäure, die nur noch einen geringen Gehalt an Gesamtsäure aufweist (im Wesentlichen Salpetersäure, Stickstoffoxide und Schwefelsäure) aus dem vorgewaschenen, DNT enthaltenen Rohgemisch die noch verbliebenen Reste an Salpetersäure, Stickstoffoxiden und Schwefelsäure derart ausgewaschen, dass der Gehalt an Mineralsäuren und Stickstoffoxiden im dem DNT enthaltenden Rohgemisch auf ein Minimum reduziert wird. Auch die im vorgewaschenen DNT verbliebene Blausäure, im Allgemeinen 20 bis 70 ppm, wird im Wesentlichen ausgewaschen. Vorzugsweise wird der zweite Waschschritt mindestens zweistufig und im Gegenstrom durchgeführt. Die Gegenstromextraktion kann bis zu m Extraktionsstufen aufweisen. Dabei wird das Extraktionsmittel der letzten der m Extraktionsstufen (WS-II-m) des zweiten Waschschritts (WS-II) zugeführt. Das zugeführte Extraktionsmittel ist im Allgemeinen Frischwasser.

Im Gegenstrom wird der zweite Waschschritt im Allgemeinen dergestalt durchgeführt, dass
I) in der ersten Extraktionsstufe (WS-II-1) des zweiten Waschschritts (WS-II) das vorgewaschene Rohgemisch enthaltend Dinitrotoluol in einen zu dieser Extraktionsstufe gehörenden ersten Mischer (M-II-1) zusammen mit im Kreis geführter Waschsäure (WSR-II-1) aus einem zu dieser Extraktionsstufe gehörenden ersten Phasentrennapparat (S-II-1) und überschüssiger Waschsäure (WSR-II-2) aus der nachfolgenden Extraktionsstufe (WS-II-2) eingespeist wird, die in dem ersten Mischer gebildete Waschemulsion in dem ersten Phasentrennapparat (S-II-1) in die Waschsäure (WSR-II-1) und einmal gewaschenes Rohgemisch (DNT-II-1) aufgetrennt, und
II) das einmal gewaschene Rohgemisch (DNT-II-1) in der nachfolgenden Extraktionsstufe (WS-II-2) des zweiten Waschschritts (WS-II) in einen zu dieser Extraktionsstufe gehörenden zweiten Mischer (M-II-2) zusammen mit der im Kreis geführten Waschsäure (WSR-II-2) aus einem zu dieser zweiten Extraktionsstufe gehörenden zweiten Phasentrennapparat (S-II-2) und, wenn der zweite Waschschritt (WS-II) mehr als 2 Extraktionsstufen umfasst, mit überschüssiger Waschsäure (WSR-I-3) aus der nachfolgenden Extraktionsstufe (WS-I-3) oder, wenn der Waschschritt genau 2 Extraktionsstufen umfasst, mit Frischwasser eingespeist wird,
wobei, wenn der zweite Waschschritt mehr als 2 Extraktionsstufen umfasst, sich weitere Extraktionsschritte entsprechend den Schritten I) und II) anschließen können.

Dabei wird der zweite Waschschritt derart durchgeführt, dass im Wesentlichen die Gesamtmenge an Nitrokresolen und Nitrophenolen und vorzugsweise bis zu 95 Gew.-% der in dem ursprünglich in dem Rohgemisch vorhandenen Nitrobenzoesäuren in dem Rohgemisch verbleiben und nicht mit der Waschsäure ausgetragen werden. Dies wird erfindungsgemäß dadurch erreicht, dass die aus der ersten Extraktionsstufe (WS-II-1) des zweiten Waschschrittes (WS-II) abgeführte Waschsäure einen pH-Wert von kleiner oder gleich 4 aufweist. In der als Abwasser ausgeschleusten, niedrig konzentrierten Waschsäure mit einem pH-Wert von 4 oder kleiner ist der Gehalt an Nitrokresolen und Nitrophenolen im Allgemeinen insgesamt kleiner als 5 ppm und der Gehalt an Nitrobenzoesäuren im Allgemeinen kleiner 50 ppm. Dieses Abwasser, das nur noch das gewaschene DNT bis zur Sättigungsgrenze gelöst enthält, kann ohne die bisher übliche und notwendige Vorbehandlung zur Entfernung der Nitrokresole und der Nitrobenzoesäuren sowie toxischer oxidativer Abbauprodukte, direkt oder nach Rückgewinnung von gelöstem DNT durch Extraktion mit Toluol und Strippung, einer Kläranlage zugeführt werden.

Vorzugsweise erfolgt in jedem der beiden Waschschritte (WS-I) und (WS-II) die Wäsche in mindestens zwei Stufen im Gegenstrom. In einer Ausführungsform umfasst der erste Waschschritt (WS-I) 2 bis 4 Extraktionsstufen (n = 2 - 4). In einer weiteren Ausführungsform umfasst der zweite Waschschritt (WS-II) 2 bis 4 Extraktionsstufen (m = 2 - 4). Es können auch mehr Extraktionsstufen, beispielsweise 5 oder 6 pro Waschschritt vorgesehen werden. In einer Ausführungsform umfasst jeder Waschschritt genau 2 Extraktionsstufen.

Die in der letzten Extraktionsstufe eines jeden Waschschrittes zuzugebende Extraktionsmittel- bzw. Frischwassermenge hängt dabei davon ab, welcher Gesamtsäuregehalt (in WS-I) bzw. welcher pH-Wert (in WS-II) in der jeweils ersten Extraktionsstufe des jeweiligen Waschschrittes (WS-I) bzw. (WS-II) vorliegen soll.

Das Volumenverhältnis von DNT enthaltendem Rohgemisch (organischer Phase) einerseits zu Waschsäure (wässriger Phase) andererseits, die in jedem Waschapparat in direktem Kontakt miteinander stehen, wird in beiden Waschschritten im Allgemeinen von 1 : 4 bis 10 : 1, bevorzugt von 1 : 3 bis 5 : 1 und besonders bevorzugt von 1 : 3 bis 2 : 1 gewählt. Je nach Phasenverhältnis und Energieeintrag bei der Dispergierung kann das Gemisch als Öl in Wasser- (O/W-Emulsion) oder als Wasser in Öl-Emulsion (W/O-Emulsion) vorliegen. Diese Phasenverhältnisse können durch Zugabe der entsprechenden Extraktionsmittelmenge zu der letzten Extraktionsstufe, bevorzugt aber, bei definierter Extraktionsmittelmenge, durch Kreisführung der Waschsäure nach Phasentrennung eingestellt werden, wobei nur der Überschuss (entsprechend der in der letzten Extraktionsstufe frisch zugeführten Extraktionsmittelmenge) in die vorhergehende Extraktionsstufe eingespeist bzw. ausgeschleust wird.

Der pH-Wert der Waschsäure, die aus der ersten Extraktionsstufe (WS-II-1) des zweiten Waschschrittes (WS II) abgezogen wird, liegt im Allgemeinen im Bereich von 0 bis 3, bevorzugt von 0,5 bis 2 und besonders bevorzugt von 0,8 bis 1,2. Der gewünschte, optimale pH-Wert in der ersten Extraktionsstufe des zweiten Waschschritts kann durch den Restgehalt an Salpetersäure des aus dem ersten Waschschritt stammenden, vorgewaschenen Rohgemischs und/oder über die in der letzten Extraktionsstufe des zweiten Waschschritts zugesetzte Frischwassermenge eingestellt werden. Alternativ dazu kann der gewünschte pH-Wert auch durch Zugabe einer Mineralsäure, beispielsweise der Waschsäure aus dem ersten Waschschritt, oder von Schwefelsäure oder bevorzugt Salpetersäure eingestellt werden.

Die Menge an Frischwasser bzw. Waschsäure, die in der letzen Extraktionsstufe des ersten Waschschrittes (WS-I) zugesetzt wird, ist so gewählt, dass die Waschsäure WSR-I-1 eine Gesamtsäurekonzentration von 10-40 Gew.-%, bevorzugt 20-30 Gew.-%, aufweist.

Die Menge an Frischwasser, die in der letzten Extraktionsstufe des zweiten Waschschrittes (WS-II) zugesetzt wird, und damit das Verhältnis DNT zu Frischwasser kann, auf Volumenbasis, von 1 : 2 bis 15 : 1, entsprechend 1,5 m³ bis 0,05 m³ Frischwasser pro t DNT, bevorzugt von 1 : 1 und 7 : 1, entsprechend 0,75 m³ bis 0,107 m³ Frischwasser pro t DNT, und besonders bevorzugt von 1 : 1 bis 2 : 1, entsprechend 0,75 m³ bis 0,38 m³ Frischwasser pro t DNT, variiert werden. In beiden Waschschritten wird bei einer Temperatur oberhalb des Schmelzpunktes von DNT, im Allgemeinen bei 60 bis 75 °C, gearbeitet.

Als Waschapparate für beide Waschschritte können beispielsweise Mixer-Settler-Apparate oder gerührte mehrstufige oder gepulste Packungs- und Siebbodenkolonnen, aber auch statische Mischer in Verbindung mit Rohrreaktoren und geeigneten Scheideapparaten eingesetzt werden. Für die Trennung der Waschdispersion aus zu waschendem DNT-haltigen Rohgemisch und Waschsäure sind sowohl statische Abscheider wie dynamische Abscheider (Zentrifugalseparatoren) geeignet.

Die aus der ersten Extraktionsstufe (WS-II-1) des zweiten Waschschritts (WS-II) ausgeschleuste Waschsäure mit einem pH-Wert von kleiner oder gleich 4, bevorzugt 0 bis 3, besonders bevorzugt 0,5 bis 2 und insbesondere 0,8 bis 1,2 weist einen Gehalt an Sulfat von maximal 100 ppm, einem Gehalt an Salpetersäure von maximal 2000 ppm und einem Gehalt an HNO₂ von maximal 50 ppm auf. Daneben enthält sie, entsprechend der Sättigungsgrenze bei der vorgegebenen Temperatur, beispielsweise 800 bis 1000 ppm isomere Dinitrotoluole. Der Gehalt an Nitrokresolen und Nitrophenolen ist im Allgemeinen kleiner als 1 ppm, der Gehalt an Nitrobenzoesäuren ist im Allgemeinen kleiner als 50 ppm.

Der Gehalt an Blausäure variiert zwischen 30-120 ppm, bevorzugt 40-120 ppm und besonders bevorzugt 50-100 ppm. Der TOC (total organic carbon) dieses Abwassers beträgt beispielsweise nur noch ca.1100 mg/l - gegenüber ca. 3000 mg/l in einem Abwasser aus der alkalischen DNT-Wäsche mit Soda. Der CSB-Gehalt (chemischer Sauerstoffbedarf) dieses Abwassers beträgt beispielsweise nur noch ca. 3000 mg O/l - gegenüber ca. 6000 mg O/l in einem Abwasser aus der alkalischen DNT-Wäsche mit Soda.

Im Schritt Extraktion/Strippung wird aus der in der Extraktionsstufe (WS-II-1) abgetrennten Waschsäure (WSR-II-1) mit einem pH kleiner gleich 4 durch Extraktion mit Toluol das in dieser Waschsäure gelöste und suspendierte DNT, im Allgemeinen 800 bis 2000 ppm, zurückgewonnen. Zusätzlich kann die Waschsäure (WSR-II-1) gemeinsam mit dem Brüdenkondensat aus der Aufkonzentrierung der Waschsäure (WRS-I-1) aus dem ersten Waschschritt (WR-I-n), wie in EP 0 737 514 beschrieben, behandelt werden. Nach Abtrennung des Extraktionsmittels von der Waschsäure (WSR-II-1) verbleiben ca. 100 bis 500 ppm an gelöstem und suspendiertem Toluol. Dieses Raffinat wird anschließend einer Strippbehandlung unterworfen, bei der neben 100 bis 500 ppm an gelöstem Extraktionsmittel auch die gelöste Blausäure, die im Mengen von beispielsweise 50 bis 100 ppm vorliegt, aus dem Abwasser bis auf einen vorgegebenen Grenzwert entfernt wird.

Die Extraktion wird bevorzugt unmittelbar nach der Abtrennung der Waschsäure (WSR-II-1) vom gewaschenen DNT im Temperaturbereich von beispielsweise 60 bis 70°C durchgeführt, um ein Abscheiden des gelösten DNT aus der mit DNT gesättigten Waschsäure zu vermeiden. Das Verhältnis Extraktionsmittel zu Waschsäure (WRS-II-1) wird so gewählt, dass eine Entfernung des DNT aus der Waschsäure bis auf den gewünschten Grenzwert mit einer möglichst geringen Anzahl von Extraktionsstufen zu erreichen ist. Das Gewichtsverhältnis von Extraktionsmittel zu behandelnder Waschsäure kann im Bereich von 1:10 bis 1:1, bevorzugt von 1:5 bis 1:3 variiert werden. Üblicherweise wird die Waschsäure (WRS-II-1) bzw. das Gemisch aus Waschsäure (WRS-II-1) und Brüdenkondensat aus der Aufkonzentrierung der Waschsäure (WRS-I-1) ein bis fünfmal, vorzugsweise ein bis dreimal extrahiert. Die Extraktion wird üblicherweise nach den bekannten Verfahren nach dem Stand der Technik für eine Flüssig/Flüssigextraktion durchgeführt, bevorzugt im Gegenstrom, wenn mehr als eine Extraktionsstufe benötigt wird. Als Extraktionsapparate können Mixer/Settler, wie z.B. in DE 1 135 425 beschrieben, oder gerührte oder gepulste Packungs- und Siebbodenkolonnen eingesetzt werden, aber auch statische Mischer in Verbindung mit geeigneten Scheideapparaten oder Kolonnen ohne Energieeintrag können verwendet werden. Das nach der Extraktion abgetrennte Toluol, das die aus der Waschsäure extrahierbaren organischen Stoffe, im wesentlichen DNT enthält, wird zusammen mit dem bei der nachfolgenden Strippung der extrahierten Waschsäure anfallenden Toluol in die Nitrierung zurückgeführt.

Die Strippung des Abwassers zur Entfernung des gelösten und suspendierten Extraktionsmittels Toluol und des bei pH ≤ 4 als Blausäure vorliegenden Cyanids kann mit Dampf als Strippgas durch Direkteinleitung oder durch Destillation (indirekt), aber auch mit Luft oder Inertgas, bevorzugt Stickstoff, unter Ausschluss von Sauerstoff durchgeführt werden. Als Strippapparate sind alle Arten von Strippkolonnen geeignet. Die Menge an Strippgas im Verhältnis des zu strippenden Abwassers wird so gewählt, dass einerseits die gewünschten Grenzwerte an Cyanid und Toluol im gestrippten Abwasser erreicht werden, und dass andererseits im Strippkondensat, nach Abtrennung von Toluol, der Gehalt an Cyanid (Blausäure) nur so hoch ist, dass eine gefahrlose Handhabung dieses blausäurehaltigen Abwassers möglich ist.

Der im Vergleich zum Abwasser im Strippkondensat höhere Cyanidgehalt kann nach dem Stand der Technik z.B. durch Zusatz von Formaldehyd gebunden werden oder durch eine elektrolytische Behandlung an geeigneten Elektroden oder durch Zugabe von Oxidationsmitteln wie Wasserstoffperoxid, Persäuren, Hypochlorid oder Ozon in ausreichender Menge soweit reduziert oder auch vollständig entfernt werden, dass das behandelte Strippkondensat mit einem Restgehalt an Blausäure entweder in die Extraktionsstufe zurückgeführt oder gefahrlos in eine Kläranlage abgegeben werden kann. Der im Strippkondensat angereicherte Cyanidgehalt kann aber auch durch eine zusätzliche Strippung des Strippkondensats mit Luft oder Stickstoff reduziert oder auch vollständig entfernt werden. Das vom überschüssigen Cyanid befreite Strippkondensat wird ebenfalls in die Extraktionsstufe zurückgeführt. Der mit Blausäure beladene Inertgasstrom wird z.B. zusammen mit dem CO enthaltenden Abgasstrom aus der Nitrierung thermisch oxidiert.

Das resultierende Abwasser nach Extraktion und Strippung mit einem pH-Wert von ≤ 4, bevorzugt 0 bis 3, besonders bevorzugt 0,5 bis 2 und insbesondere 0,8 bis 1,2 weist im Allgemeinen einen Gehalt an Sulfat von maximal 100 ppm, einem Gehalt an Salpetersäure von maximal 2000 ppm und einem Gehalt an HNO₂ von maximal 50 ppm auf. Daneben enthält es weniger als 50 ppm, bevorzugt weniger als 20 ppm und besonders bevorzugt weniger als 1 ppm an isomeren Dinitrotoluolen. Der Gehalt an Nitrokresolen und Nitrophenolen ist im Allgemeinen kleiner als 1 ppm, der Gehalt an Nitrobenzoesäuren ist im Allgemeinen kleiner als 50 ppm. Der Gehalt an freier Blausäure ist kleiner als 1 ppm, bevorzugt kleiner 0,5 ppm und besonders bevorzugt kleiner 0,2 ppm. Der TOC (total organic carbon) dieses Abwassers beträgt beispielsweise nur noch ca. 500 bis 600 mg/l - gegenüber ca. 1100 mg/l in der Waschsäure (WRS-II-1). Der CSB-Gehalt (chemischer Sauerstoffbedarf) dieses Abwassers beträgt beispielsweise nur noch ca. 1500 mg O/l - gegenüber ca. 3000 mg O/l in der Waschsäure (WRS-II-1) vor dem Schritt Extraktion/Strippung.

Die so vorbehandelte Waschsäure (WRS-II-1) wie auch ein so behandeltes Gemisch aus Waschsäure (WRS-II-1) und aus Brüdenkondensat der Waschsäure-Aufkonzentrierung (WRS-I-1) erfüllt, nach Neutralisation und Durchgang durch eine biologische Behandlungsstufe in einer Kläranlage, im Allgemeinen alle Anforderungen der Abwasserverordnung der Bundesrepublik Deutschland bezüglich Toxizität einschließlich Gentoxizität, bestimmt nach DIN EN ISO 38415 T6 (Fisch), 11348-2 (Leuchtbakterien), 38412 L30 (Daphnien), 38412 L33 (Algen) und DIN EN ISO 9888 (Umu Test, Gentoxizität).

Aus dem zweiten Waschschritt (WS-II) wird aus der letzten Extraktionsstufe (WR-II-m) ein DNT abgezogen, welches eine Restacidität von im Allgemeinen maximal 300 ppm Salpetersäure, einen Gehalt an Blausäure von im Allgemeinen maximal 50 ppm, bevorzugt maximal 25 ppm und besonders bevorzugt 10 ppm und ganz besonders bevorzugt maximal 1 ppm aufweist. Der Distickstoffmonoxidgehalt (N₂O) beträgt im Allgemeinen maximal 200 ppm, bevorzugt maximal 50 und besonders bevorzugt maximal 25 ppm, der CO-Gehalt im Allgemeinen maximal 400 ppm, bevorzugt maximal 200 ppm und besonders bevorzugt maximal 50 ppm CO, der Gehalt an Nitrokresolen und Nitrophenolen im Allgemeinen maximal 800 ppm, der Gehalt an Nitrobenzoesäuren im Allgemeinen maximal 600 ppm (Dinitro- und Mononitrobenzoesäuren), der Restgehalt an Salpetersäure im Allgemeinen maximal 300 ppm und der Sulfatgehalt im Allgemeinen maximal 3 ppm . Der pH-Wert beträgt im Allgemeinen von 2 bis 4. Dieses DNT und die darin gelösten Nitrokresole, Nitrophenole und Nitrobenzoesäuren können entsprechend dem Stand der Technik vollständig und problemlos zu Toluylendiamin (TDA) und den entsprechenden Aminokresolen, Aminophenolen und Aminobenzoesäuren (Diamino- und Monoaminobenzoesäuren) hydriert werden. Die Aminokresole, Aminophenole und Aminobenzoesäuren verbleiben bei der TDA-Destillation im Destillationsrückstand und werden zusammen mit diesem entsorgt.

Ein weiterer Vorteil ist die höhere thermische Stabilität des erfindungsgemäß einer sauren Wäsche unterworfenen DNT. So wird die Temperatur, bei der die thermische Zersetzung beginnt, bei einem nur sauer gewaschenen DNT, welches beispielsweise 620 ppm an Nitrophenolen und Nitrokresolen und 460 ppm an Nitrobenzoesäuren enthält, im Vergleich zu einem DNT, das nach dem Stand der Technik in Gegenwart von Soda gewaschen wurde und beispielsweise weniger als 20 ppm an Nitrophenolen und Nitrokresolen enthält, um ca. 20 °C angehoben.

Zum Nachweis des im Verlauf des während der Nitrierung von Toluol zu DNT gebildeten Cyanids in den Waschsäuren aus (WS-I) und (WS-II) kann nach der in DIN 38405-D13 beschriebenen Methode gearbeitet werden. Es ist darauf zu achten, dass durch Verdünnen der Probe vor Durchführung der Analyse die Grenzkonzentration der Störstoffe eingehalten werden. Das Gleiche gilt für die handelsüblichen Küvettentests.

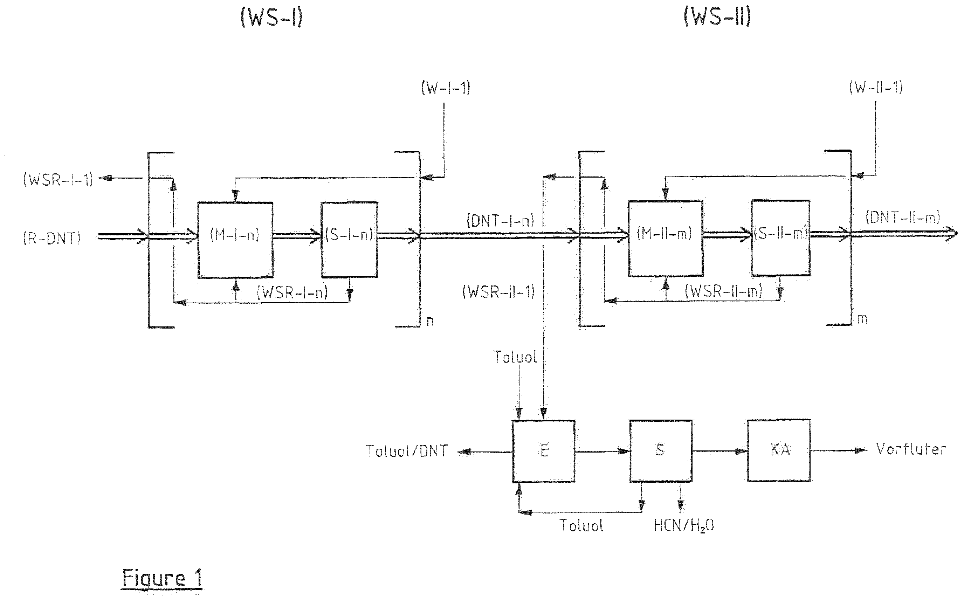

Figur 1 zeigt eine schematische Darstellung einer Wäsche von Roh-DNT nach dem erfindungsgemäßen Verfahren gemäß einer bevorzugten Ausführungsform der Erfindung.

Das rohe DNT (R-DNT) aus der Nitrierung nach Abtrennung der DNT-Endsäure wird im Waschschritt I (WS-I) in der ersten Extraktionsstufe (WS-I-1) (n=1) zusammen mit der im Kreis geführten Waschsäure (WSR-I-1) aus dem Phasentrennapparat (S-I-1) und zusammen mit der überschüssigen Waschsäure (WSR-I-2) aus der nachfolgenden Extraktionsstufe (WS-I-2) in den Mischer (M-I-1) so eingespeist, dass sich das vorgegebene Phasenverhältnis Waschsäure zu DNT einstellt. Nach Phasentrennung der Waschemulsion in dem zur Extraktionsstufe gehörenden Phasentrennapparat (S-I-1) wird die abgetrennte Waschsäure (WSR-I-1) in den zu dieser Stufe gehörenden Mischer (M-I-1) zurückgeführt. Die überschüssige Waschsäure (WSR-I-1) wird direkt oder nach Aufkonzentrierung in die Nitrierung zurückgeführt.

Das im Scheider (S-I-1) abgetrennte, einmal gewaschene DNT (DNT-I-1) wird in die nachfolgende Extraktionsstufe (WS-I-2)(n=2) zusammen mit der im Kreis geführten Waschsäure (WSR-I-2) aus dem Phasentrennapparat (S-I-2) zusammen mit der überschüssigen Waschsäure (WSR-I-3) aus der nachfolgenden Extraktionsstufe (WS-I-3) in den Mischer (M-I-2) so eingespeist, dass sich das vorgegebene Phasenverhältnis Waschsäure zu DNT einstellt. Nach Phasentrennung der Waschemulsion in dem zu dieser Extraktionsstufe gehörenden Phasentrennapparat (S-I-2) wird die abgetrennte Waschsäure (WSR-I-2) in den zur Stufe gehörenden Mischer (M-I-2) zurückgeführt. Die überschüssige Waschsäure (MSR-I-2) wird in die vorgeschaltete Extraktionsstufe (WS-I-1) eingespeist. Das im Phasentrennapparat (S-I-2) abgetrennte, zweimal gewaschene DNT (DNT-I-2) wird in die nächste Extraktionsstufe (WS-I-3)(n=3) für eine dritte Wäsche transferiert. Die Anzahl der Extraktionsstufen kann vorzugsweise bis zu vier (n=4) betragen. In die letzte Extraktionsstufe (WS-I-4) wird als Waschmedium (W-I-1) Frischwasser oder bevorzugt das Brüdenkondensat aus der Aufkonzentrierung der Waschsäure aus der Waschstufe (WS-I-1) in der Menge eingespeist, dass die aus (S-I-1) abgetrennte Waschsäure einen Gesamtsäuregehalt von 20 bis 40% aufweist.

Das vorgewaschene DNT (DNT-I-n) aus Waschschritt (WS-I) wird im Waschschritt (WS-II), in der ersten Extraktionsstufe (WS-II-1) (m=1) zusammen mit der im Kreis geführten Waschsäure (WSR-II-1) aus dem Phasentrennapparat (S-II-1) zusammen mit der überschüssigen Waschsäure (WSR-II-2) aus der nachfolgenden Extraktionsstufe (WS-II-2) in den Mischer (M-II-1) so eingespeist, dass sich das vorgegebene Phasenverhältnis Waschsäure zu DNT einstellt. Nach Phasentrennung der Waschemulsion in dem zur Waschstufe gehörenden Phasentrennapparat (S-II-1) wird die abgetrennte Waschsäure (WSR-II-1) in den zu dieser Stufe gehörenden Mischer (M-II-1) zurückgeführt. Die überschüssige Waschsäure (WSR-II-1) wird anschließend in den erfindungsgemäßen Schritten Extraktion und Strippung zur Rückgewinnung des darin gelösten Produktes (DNT) weiterbehandelt.

Das im Phasentrennapparat (S-II-1) abgetrennte, einmal gewaschene DNT (DNT-II-1) wird in die nachfolgende Extraktionsstufe (WS-II-2) (m=2) zusammen mit der im Kreis geführten Waschsäure (WSR-II-2) aus dem Phasentrennapparat (S-II-2) und der überschüssigen Waschsäure (WSR-II-3) aus der nachfolgenden Waschstufe (WS-II-3) in den Mischer (M-II-2) so eingespeist, dass sich das vorgegebene Phasenverhältnis Waschsäure zu DNT einstellt. Nach Phasentrennung der Waschemulsion in dem zu dieser Extraktionsstufe gehörenden Phasentrennapparat (S-II-2) wird die abgetrennte Waschsäure (WSR-II-2) in den zu dieser Stufe gehörenden Mischer (M-II-2) zurückgeführt. Die überschüssige Waschsäure (MSR-II-2) wird in die vorgeschaltete Extraktionsstufe (WS-II-1) eingespeist. Das im Phasentrennapparat (S-II-2) abgetrennte, zweimal gewaschene DNT (DNT-II-2) wird in die nächste Extraktionsstufe (WS-II-3)(m=3) für eine dritte Wäsche transferiert. Die Anzahl der Extraktionsstufen kann vorzugsweise bis zu sechs (m=6) betragen. In die letzte Extraktionsstufe (WS-II-6) wird als Waschmedium (W-II-1) Frischwasser in der Menge eingespeist, dass die aus (S-II-1) abgetrennte Waschsäure einen pH-Wert von bevorzugt 0 bis 3 aufweist. Durch Zusatz von Salpetersäure oder Waschsäure (WSR-I-1) kann der pH-Wert in der ersten Extraktionsstufe (WS-II-1) zusätzlich auf den optimalen Betriebswert nachjustiert werden.

Die überschüssige Waschsäure (WSR-II-1) aus der ersten Extraktionsstufe (WS-II-1) wird anschließend im Extraktionsschritt (E) mit Toluol in einem Extraktionsapparat behandelt. Nach Phasentrennung des Extraktionsgemisches Toluol/(WSR-II-1) wird dieses Toluol zusammen mit dem gelösten DNT in die Nitrierung eingespeist. Die noch gelöstes und Spuren von suspendiertem Toluol und das Cyanid enthaltende Waschsäure wird im Schritt Strippung (S) von diesem Toluol zusammen mit dem Cyanid befreit. Die von Toluol und Cyanid befreite Waschsäure wird nach Neutralisation direkt in eine biologische Behandlungsstufe einer Kläranlage abgegeben. Das bei der Strippung abgetrennte Toluol wird in die Extraktion zurückgeführt. Die im Strippkondensat angereicherte Blausäure kann z.B. durch Behandlung mit Oxidationsmitteln nach dem Stand der Technik zerstört werden.

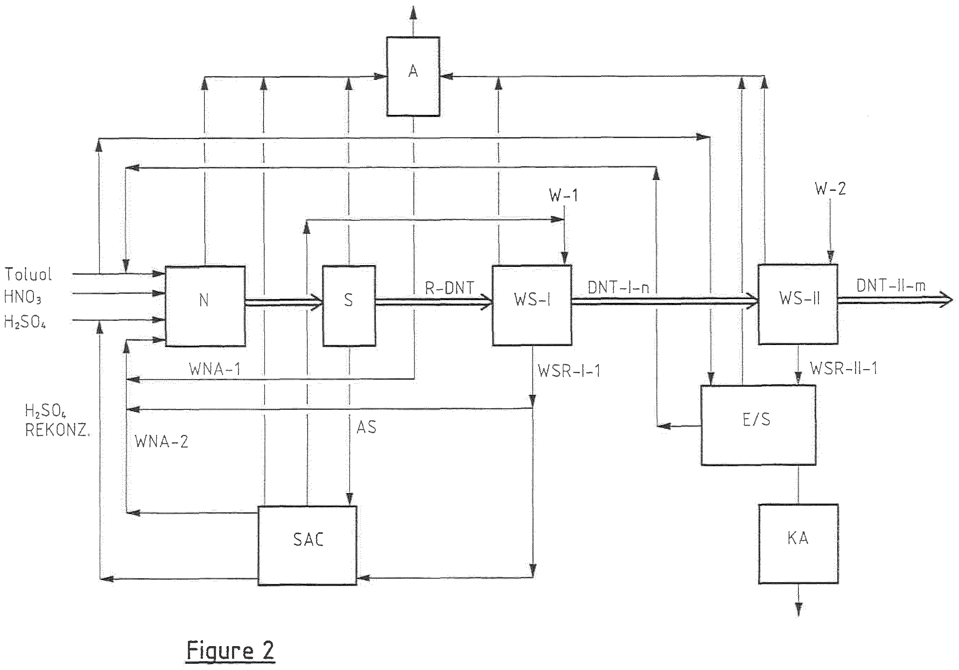

Figur 2 zeigt eine schematische Darstellung einer erfindungsgemäßen Produktionsanlage zur Nitrierung von Toluol zu DNT mit nachfolgender erfindungsgemäßer Wäsche des Roh-DNT in zwei Schritten gemäß einer bevorzugten Ausführungsform der Erfindung.

Das in der Nitriereinheit (N) durch eine zweistufige kontinuierliche isotherme oder adiabatische Nitrierung von Toluol in einem Gemisch aus Schwefel- und Salpetersäure im Gegenstrom gebildete Dinitrotoluol-Isomerengemisch wird nach Trennung der Nitrieremulsion im Phasentrennapparat (Scheider (S)) in eine erfindungsgemäße Wäsche mit zwei Waschschritten (WS-I) und (WS-II) eingespeist. In Waschschritt (WS-I) werden die im rohen DNT gelösten Mineralsäuren Schwefelsäure, Salpetersäure und Stickstoffoxide ausgewaschen. Die in diesem Waschschritt aus der ersten Waschstufe (WS-I-1) abgetrennte Waschsäure (WSR-I-1) mit einem Gesamtsäuregehalt von 10 - 40 Gew.-% wird direkt in die Nitriereinheit (N) oder nach weiterem Aufkonzentrieren als (WNA-2) in die Nitrierung zurückgeführt. Das Brüdenkondensat aus der Aufkonzentrierung der Waschsäure (WRS-I-1) und/oder der Nitrierendsäure wird in den Waschschritt (WS-I) als Waschwasser (W-1) zurückgeführt.

Im zweiten Waschschritt (WS-II) werden die im vorgewaschenen DNT aus (WS-I) noch verbliebenen Reste an Mineralsäuren derart ausgewaschen, dass eine Waschsäure (WSR-II-1) mit einem pH-Wert im Bereich von vorzugsweise 0 bis 3 resultiert. Zur Ergänzung der abgetrennten Waschsäuremenge wird in (WS-II) Frischwasser zugesetzt. Diese Waschsäure aus Waschschritt II (WS-II) wird zur Rückgewinnung des darin gelösten DNT und Entfernung des Cyanid im Verfahrensschritt Extraktion/Strippung (E/S) so behandelt, dass sie, nach Neutralisation, direkt in eine biologische Nachbehandlungsstufe in einer Kläranlage (KA) eingeleitet werden kann.

Die in der Scheideeinheit (S) erhaltenen Nitrierendsäure wird in einer Aufkonzentrieranlage für Schwefelsäure (SAC) wieder zu Schwefelsäure (bis zu 96%) aufkonzentriert und in die Nitrierung zurückgeführt. Die in der Abgasbehandlung (A) aus den gesammelten, Stickstoffoxide enthaltenden Abgasen aus Nitrierung und Wäsche zurück gewonnene Salpetersäure (WNA-1) wird zusammen mit der aus Waschschritt (WS-I) anfallenden Waschsäure (WSR-I-1) oder, nach Aufkonzentration, als (WNA-2) gleichfalls in die Nitrierung zurückgeführt.

Die vorliegende Erfindung wird an Hand des nachfolgenden Ausführungsbeispiels verdeutlicht.

### Beispiele

### Beispiel 1 (nach dem Stand der Technik)

4500 kg/h DNT mit einem Restgehalt an 0,94 Gew.-% Schwefelsäure, 1,43 Gew.-% Salpetersäure und 1,15 Gew.-% Stickstoffdioxid wurden nach Abtrennung der Nitrierendsäure in einer sauren Wäsche (Waschschritt I) in 2 Stufen im Gegenstrom gewaschen. In der ersten Extraktionsstufe wurde mit einer Waschsäure mit 7,58 Gew.-% Schwefelsäure, 19,52 Gew.-% an Salpetersäure und ca. 0,45 Gew.-% an Stickstoffoxiden (als HNO₂) im Phasenverhältnis DNT zu Waschsäure von 1 : 1 gewaschen. In der zweiten Extraktionsstufe des Waschschrittes I wurde gleichfalls im Phasenverhältnis 1 : 1 mit einer Waschsäure mit maximal 0,5 Gew.-% Schwefelsäure, ca. 6 Gew.-% Salpetersäure und ca. 0,15 Gew.-% an Stickstoffoxiden gewaschen. Die Menge des zugesetzten Wassers bzw. Kondensates aus der weiteren Aufkonzentrierung der Waschsäure (im vorliegenden Beispiel ca. 450 l/h), das auf der letzten Extraktionsstufe eingespeist wurde, ist so gewählt, dass die Konzentration der Waschsäure in der ersten Extraktionsstufe der sauren Wäsche, im Kontakt mit dem zu waschenden Roh-DNT, die festgelegte Säurestärke und Dichte nicht überschreitet. Zur Aufrechterhaltung des vorgegebenen Phasenverhältnisses wurde die im Scheider (Phasentrennapparat) der jeweiligen Extraktionsstufe abgetrennte Waschsäure im Kreis geführt und nur der Überschuss wurde in die vorgelagerte Waschstufe transferiert bzw. ausgeschleust.

Die aus der sauren Wäsche ausgeschleuste Waschsäure mit einem Gesamtsäuregehalt von 27,55 Gew.-% wurde nach weiterer Aufkonzentrierung in die Nitrierung zurückgeführt.

Das DNT aus der sauren Wäsche, das fast frei von den Mineralsäuren war, wurde in der alkalischen Wäsche von den Resten der im vorgewaschenen DNT noch gelösten Mineralsäuren, vor allem Salpetersäure und Nitrose, bzw. mitgerissenen Waschsäure, den Nitrophenolen und Nitrokresolen, Nitrobenzoesäuren und allen sonstigen starken und schwachen Sauren aus der Oxidation der Nitro- und Dinitrokresole in der DNT-Stufe, wie z.B. Oxalsäure, Essigsäure, Ameisensäure Blausäure, Kohlenstoffdioxid etc. befreit.

Ca. 4350 kg an vorgewaschenem DNT aus Waschschritt I (saure Wäsche) mit einem Restgehalt von maximal 100 ppm an Schwefelsäure und maximal 3000 ppm an Salpetersäure/Stickstoffoxiden wurden in einem alkalischen Waschschritt (Alkaliwäsche) in Gegenwart einer Base (Soda) im Phasenverhältnis 1 : 1 in einer Stufe gewaschen, wobei sich ein pH von 9-10 einstellt. Nach Phasentrennung der Waschemulsion aus der Alkaliwäsche wurde das abgetrennte DNT noch in einer einstufigen Neutralwäsche, ebenfalls im Phasenverhältnis 1 : 1, von den mitgerissenen Spuren aus der Alkaliwäsche durch Zugabe von Frischwasser (im vorliegendem Falle 2800 l/h) gewaschen. Nach Phasentrennung wird das in der Neutralwäsche abgetrennte Waschwasser in die Alkaliwäsche als Waschwasser eingespeist. Zur Aufrechterhaltung der vorgegebenen Phasenverhältnisse in den beiden Waschschritten (Alkaliwäsche und Neutralwäsche) wurde das im Scheider der jeweiligen Waschstufe abgetrennte Waschwasser im Kreis geführt und nur der Überschuss wurde von der Neutralwäsche in die Alkaliwäsche transferiert bzw. aus der Alkaliwäsche ausgeschleust.

Die aus der Alkaliwäsche ausgeschleuste alkalische Waschlauge mit einem pH von 9-10, einem Gehalt von 40 ppm Sulfat, 580 ppm Nitrat, 2500 ppm Nitrit, 990 ppm DNT und 740 ppm Trinitrokresole wurde direkt in eine Thermolyse bei 290°C und 90 bar zum Abbau der Nitrophenole und Nitrokresole sowie weiterer Nitroverbindungen und des gelösten DNT eingespeist. Das aus der Thermolyse ablaufende Wasser, in dem die Nitrophenole und Nitrokresole, Nitrobenzoesäuren, Blausäure und das gelösten DNT zersetzt wurden, wurde vor Abgabe in den Vorfluter noch einer biologischen Nachbehandlung in einer Kläranlage unterworfen.

### Beispiel 2 (erfindungsgemäß)

4500 kg/h DNT mit einem Restgehalt an 0,94 Gew.-% Schwefelsäure, 1,43 Gew.-% Salpetersäure, 1,15 Gew.-% Stickstoffdioxid und im Mittel 70 ppm an Blausäure wurden nach Abtrennung der Nitrierendsäure im Waschschritt I (WS-I) in 2 Stufen im Gegenstrom gewaschen. In der ersten Extraktionsstufe (WS-I-1) wurde mit einer Waschsäure mit 7,58 Gew.-% Schwefelsäure, 19,52 Gew.-% Salpetersäure, ca. 0,45 Gew.-% an Stickstoffoxiden (als HNO₂) und im Mittel 250 ppm an Blausäure im Phasenverhältnis DNT zu Waschsäure von 1 : 1 gewaschen. In der zweiten Extraktionsstufe (WS-I-2) des Waschschrittes I wurde gleichfalls im Phasenverhältnis 1 : 1 mit einer Waschsäure mit maximal 0,5 Gew.-% Schwefelsäure, ca. 6 Gew.-% Salpetersäure, ca. 0,15 Gew.-% Stickstoffoxiden und im Mittel 150 ppm an Blausäure (Brüdenkondensat) gewaschen. Die Menge des zugesetzten Wassers bzw. Kondensates aus der weiteren Aufkonzentrierung der Waschsäure (im vorliegenden Beispiel ca. 450 l/h), das in die letzte Extraktionsstufe eingespeist wurde, ist so gewählt, dass die Konzentration der Waschsäure in der ersten Extraktionsstufe (WS-I-1) im Kontakt mit dem zu waschenden Roh-DNT die festgelegte Säurestärke und Dichte nicht überschreitet. Zur Aufrechterhaltung des vorgegebenen Phasenverhältnisses wurde die im Scheider (Phasentrennapparat) der jeweiligen Extraktionsstufe abgetrennte Waschsäure im Kreis geführt und nur der Überschuss wurde in die vorgelagerte Waschstufe transferiert bzw. ausgeschleust.

Die im Waschschritt (WS-I) ausgeschleuste Waschsäure mit einem Gesamtsäuregehalt von *27,55* Gew.-% wurde nach weiterer Aufkonzentrierung in die Nitrierung zurückgeführt.

Das im Waschschritt (WS-I) von den Mineralsäuren fast vollständig befreite, vorgewaschene DNT wurde in Waschschritt II (WS-II) von den Resten der noch im DNT gelösten Mineralsäuren, vor allem Salpetersäure und Nitrose und Blausäure bzw. mitgerissenen Waschsäure aus Waschschritt I (WS-I) befreit.

Ca. 4350 kg an vorgewaschenem DNT aus Waschschritt I (WS-I) mit einem Restgehalt von maximal 100 ppm Schwefelsäure, maximal 3000 ppm Salpetersäure/Stickstoffoxide und im Mittel 50 ppm an Blausäure wurden in zwei Stufen im Gegenstrom gewaschen. In der ersten Extraktionsstufe des Waschschritts II (WS-II) wurde mit einer Waschsäure mit einem pH von 0 bis 3, besonders bevorzugt von 0,8 bis 1,2, mit einem Phasenverhältnis DNT : Waschsäure von 1 : 1 gewaschen. In der zweiten Extraktionsstufe des Waschschrittes II (WS-II) wurde gleichfalls im Phasenverhältnis 1 : 1 mit einer Waschsäure, die nur noch Spuren an Mineralsäure, vor allen Salpetersäure aufwies, gewaschen. Die Menge des zugesetzten Frischwassers (im vorliegenden Beispiel ca. 2800 l/h), das in die letzte Extraktionsstufe (WS-II-n) (n=2) eingespeist wurde, wurde so gewählt, dass für den pH-Wert in der ersten Extraktionsstufe (WS-II-1), in der das DNT aus dem Waschschritt I (WS-I) extrahiert wird, der festgelegte optimale Wert für den pH im Bereich von 0,8 bis 1,2 eingehalten wurde. Zur Aufrechterhaltung der vorgegebenen Phasenverhältnisse in den Extraktionsstufen wurde die im Scheider der jeweiligen Extraktionsstufe abgetrennte Waschsäure im Kreis geführt, und nur der Überschuss wurde in die vorgelagerte Stufe transferiert bzw. ausgeschleust.

Die in Waschschritt II (WS-II) aus der ersten Extraktionsstufe (WS-II-1) ausgeschleuste Waschsäure (ca. 2800 I) mit einem pH-Wert von 1,0, einem Gehalt von 85 ppm Schwefelsäure, 1800 ppm Salpetersäure, 40 ppm Stickstoffoxide, 980 ppm DNT, kleiner 1 ppm Trinitrokresole, weniger als 10 ppm Nitrobenzoesäuren und im Mittel 80 ppm an Blausäure wurde mit Toluol bei einem Phasenverhältnis (Volumen) von Toluol zu Wasser von 1 : 3 mehrstufig extrahiert. Nach Phasentrennung wurde das Toluol-Extrakt zusammen mit dem DNT in die Nitrierung zurückgeführt. Die Waschsäure (Raffinat) nach der Extraktion mit Toluol enthielt noch weniger als 1 ppm an DNT, im Mittel bis zu 500 ppm an Toluol und ca. 80 ppm an Blausäure. In einer mehrstufigen Strippung mit Dampf, gegebenenfalls noch unterstützt durch einen Inertgasstrom, wurden Toluol und Blausäure abgetrennt. Beide Stoffe lagen im Kondensat angereichert vor. Das abgetrennte Toluol wurde in die Extraktion zurückgeführt. Das im Stripp-Kondensat angereicherte Cyanid (z.B. um einen Faktor 10) wurde durch Behandlung mit Oxidationsmitteln nach dem Stand der Technik abgebaut. Dieses so behandelte Kondensat, das im Wesentlichen frei von Cyanid war, wurde in die Extraktion zurückgeführt. Die Waschsäure, die frei von Toluol und Cyanid (max. 0,2 ppm) war, wurde nach Neutralisation auf einen pH von 7-8 direkt in eine biologische Nachbehandlung eingespeist. Das nach dieser biologischen Behandlung erhaltene Abwasser erfüllte alle Anforderungen an die Toxizität, die in der Abwasserverordnung der Bundesrepublik Deutschland festgelegt sind.

## Patentansprüche

1. Verfahren zur Wäsche eines bei der Nitrierung von Toluol nach Abtrennung der Nitriersäure anfallenden Rohgemischs enthaltend Dinitrotoluol, Salpetersäure, Stickstoffoxide und Schwefelsäure umfassend zwei Waschschritte (WS-I) und (WS-II), wobei
i) in einem ersten Waschschritt (WS-I) das Rohgemisch in einer mindestens eine Extraktionsstufe umfassenden Wäsche mit einer Salpetersäure, Stickstoffoxide und Schwefelsäure enthaltenden Waschsäure I extrahiert wird, wobei die aus der ersten Extraktionsstufe (WS-I-1) des ersten Waschschrittes (WS-I) abgeführte Waschsäure einen Gesamtsäuregehalt von 10 bis 40 Gew.-% und einen Gehalt an 80 bis 350 ppm Blausäure aufweist, wobei ein vorgewaschenes Rohgemisch erhalten wird,
ii) in einem zweiten Waschschritt (WS-II) das vorgewaschene Rohgemisch enthaltend Dinitrotoluol in einer mindestens eine, bevorzugt mindestens 2 Extraktionsstufe umfassenden Wäsche mit einer Waschsäure II extrahiert wird, wobei die aus der ersten Extraktionsstufe (WS-II-1) des zweiten Waschschrittes (WS-II) abgeführte Waschsäure einen pH-Wert von kleiner oder gleich 4 und einen Gehalt an Sulfat von maximal 100 ppm, einen Gehalt an Salpetersäure von maximal 2000 ppm, einen Gehalt an Stickstoffoxiden (berechnet als HNO₂) von maximal 50 ppm und einen Gehalt an Blausäure von maximal 120 ppm aufweist, wobei ein von Salpetersäure, Schwefelsäure, Stickstoffoxiden und Blausäure im wesentlichen freies, Dinitrotoluol enthaltendes Gemisch mit einem Gehalt von maximal 300 ppm Salpetersäure und Stickoxiden, maximal 3 ppm Sulfat und maximal 50 ppm Blausäure erhalten wird.

2. Verfahren nach Anspruch 1, **dadurch gekennzeichnet, dass** der erste Waschschritt (WS-I) 2 bis 4 Extraktionsstufen umfasst und im Gegenstrom durchgeführt wird.

3. Verfahren nach Anspruch 1 oder 2, **dadurch gekennzeichnet, dass** der zweite Waschschritt (WS-II) 2 bis 6 Extraktionsstufen umfasst und im Gegenstrom durchgeführt wird.

4. Verfahren nach einem der Ansprüche 1 bis 3, **dadurch gekennzeichnet, dass** die aus der ersten Extraktionsstufe (WS-II-1) des zweiten Waschschrittes (WS-II) abgeführte Waschsäure einen pH-Wert von 0 bis 3 aufweist.

5. Verfahren nach einem der Ansprüche 1 bis 4, **dadurch gekennzeichnet, dass** die aus der ersten Extraktionsstufe (WS-II-1) des zweiten Waschschrittes (WS-II) abgeführte Waschsäure einen pH-Wert von 0,5 bis 2 aufweist.

6. Verfahren nach einem der Ansprüche 1 bis 5, **dadurch gekennzeichnet, dass** der letzten Extraktionsstufe (WS-I-n) des ersten Waschschrittes (WS-I) Wasser oder eine Waschsäure mit einem Gesamtsäuregehalt von 0,2 bis 1,5 Gew.-% zugeführt wird.

7. Verfahren nach einem der Ansprüche 1 bis 6, **dadurch gekennzeichnet, dass** der letzten Extraktionsstufe (WS-II-n) des zweiten Waschschrittes (WS-II) Wasser zugeführt wird.

8. Verfahren nach einem der Ansprüche 1 bis 7, **dadurch gekennzeichnet, dass**
I) in der ersten Extraktionsstufe (WS-I-1) des ersten Waschschritts (WS-I) das Rohgemisch enthaltend Dinitrotoluol, Salpetersäure, Stickstoffoxide und Schwefelsäure in einen ersten Mischer (M-I-1) zusammen mit im Kreis geführter Waschsäure (WSR-I-1) aus einem zu dieser ersten Extraktionsstufe gehörenden ersten Phasentrennapparats (S-I-1) und überschüssiger Waschsäure (WSR-I-2) aus der nachfolgenden Extraktionsstufe (WS-I-2) eingespeist wird, die in dem Mischer gebildete Waschemulsion in dem ersten Phasentrennapparat (S-I-1) in die Waschsäure (WSR-I-1) und einmal gewaschenes Rohgemisch (DNT-I-1) aufgetrennt, und
II) das einmal gewaschene Rohgemisch (DNT-I-1) in der nachfolgenden Extraktionsstufe (WS-I-2) des ersten Waschschritts (WS-I) in einen zu dieser Extraktionsstufe gehörenden zweiten Mischer (M-I-2) zusammen mit der im Kreis geführten Waschsäure (WSR-I-2) aus einem zu dieser Extraktionsstufe gehörenden zweiten Phasentrennapparat (S-I-2) und, wenn der erste Waschschritt (WS-I) mehr als 2 Extraktionsstufen bis (WS-I-n) umfasst, mit überschüssiger Waschsäure (WSR-I-3) (WSR-In) aus der nachfolgenden Extraktionsstufe (WS-I-3) bis (WS-I-n) oder, wenn der erste Waschschritt genau 2 Extraktionsstufen umfasst, mit Frischwasser oder Waschsäure (W-I-1) eingespeist wird.

9. Verfahren nach einem der Ansprüche 1 bis 8, **dadurch gekennzeichnet, dass**
I) in der ersten Extraktionsstufe (WS-II-1) des zweiten Waschschritts (WS-II) das vorgewaschene Rohgemisch enthaltend Dinitrotoluol in einen zu dieser Extraktionsstufe gehörenden ersten Mischer (M-II-1) zusammen mit im Kreis geführter Waschsäure (WSR-II-1) aus einem zu dieser Extraktionsstufe gehörenden ersten Phasentrennapparat (S-II-1) und überschüssiger Waschsäure (WSR-II-2) aus der nachfolgenden Extraktionsstufe (WS-II-2) eingespeist wird, die in dem ersten Mischer gebildete Waschemulsion in dem ersten Phasentrennapparat (S-II-1) in die Waschsäure (WSR-II-1) und einmal gewaschenes Rohgemisch (DNT-II-1) aufgetrennt, und
II) das einmal gewaschene Rohgemisch (DNT-II-1) in der nachfolgenden Extraktionsstufe (WS-II-2) des zweiten Waschschritts (WS-II) in einen zu dieser Extraktionsstufe gehörenden zweiten Mischer (M-II-2) zusammen mit der im Kreis geführten Waschsäure (WSR-II-2) aus einem zu dieser zweiten Extraktionsstufe gehörenden zweiten Phasentrennapparat (S-II-2) und, wenn der zweite Waschschritt (WS-II) mehr als 2 Extraktionsstufen bis (WS-II-m) umfasst, mit überschüssiger Waschsäure (WSR-II-3) bis (WSR-II-m) aus der nachfolgenden Extraktionsstufe (WS-II-3) bis (WS-II-m) oder, wenn der Waschschritt genau 2 Extraktionsstufen umfasst, mit Frischwasser (W-II-1) eingespeist wird.

10. Verfahren nach einem der Ansprüche 1-9, **dadurch gekennzeichnet, dass** das Volumenverhältnis zwischen Dinitrotoluol enthaltendem Rohgemisch und Waschsäure in den Extraktionsstufen der Waschschritte (W-I) und (W-II) von 1 : 4 bis 10 : 1 beträgt.

11. Verfahren nach einem der Ansprüche 1-10, **dadurch gekennzeichnet, dass** das Volumenverhältnis von Dinitrotoluol enthaltendem Rohgemisch zu Frischwasser in der letzten Extraktionsstufe (W-II-m) des zweiten Waschschrittes (W-II) von 1 : 2 bis 15 : 1 beträgt.

12. Verfahren nach einem der Ansprüche 1 bis 11, **dadurch gekennzeichnet, dass** aus der letzten Extraktionsstufe (WS-II-m) des zweiten Waschschritts (WS-II) ein Dinitrotoluol enthaltendes Gemisch abgezogen wird, welches insgesamt maximal 800 ppm Nitrokresole und Nitrophenolen, maximal 600 ppm Nitrobenzoesäuren, maximal 300 ppm Salpetersäure, maximal 50 ppm Blausäure und maximal 3 ppm Sulfat aufweist.

13. Verfahren nach einem der Ansprüche 1 bis 12, **dadurch gekennzeichnet, dass** die aus der ersten Extraktionsstufe (WS-II-1) des zweiten Waschschritts (WS-II) abgeführte Waschsäure einen Gehalt an Nitrokresolen und Nitrophenolen von insgesamt kleiner als 5 ppm und einen Gehalt an Nitrobenzoesäuren von kleiner als 50 ppm aufweist.

14. Verfahren nach Anspruch 12 oder 13, **dadurch gekennzeichnet, dass** die aus der ersten Extraktionsstufe (WS-II-1) des zweiten Waschschritts (WS-II) abgeführte Waschsäure nach Abtrennung von darin gelöstem Dinitrotoluol durch Extraktion und Abtrennung des Extraktionsmittels und nach Neutralisation ohne weitere Vorbehandlung der biologischen Behandlungsstufe einer Kläranlage zugeführt wird.

15. Verfahren nach Anspruch 14, **dadurch gekennzeichnet, dass** die aus der ersten Extraktionsstufe (WS-II-1) des zweiten Waschschritts (WS-II) abgetrennte Waschsäure mit Toluol extrahiert und die extrahierte Waschsäure mit einem Strippgas gestrippt wird, wobei ein Abwasser mit einem Blausäuregehalt von kleiner 1 ppm erhalten wird.

## Claims

1. A process for scrubbing a crude mixture which is obtained in the nitration of toluene after separating off the nitrating acid and comprises dinitrotoluene, nitric acid, nitrogen oxides and sulfuric acid, which comprises two scrubbing steps (WS-I) and (WS-II), wherein
i) in a first scrubbing step (WS-I), the crude mixture is extracted with a scrubbing acid I comprising nitric acid, nitrogen oxides and sulfuric acid in a scrub comprising at least one extraction stage, where the scrubbing acid discharged from the first extraction stage (WS-I-1) of the first scrubbing step (WS-I) has a total acid content of from 10 to 40% by weight and a content of from 80 to 350 ppm of hydrocyanic acid, giving a prescrubbed crude mixture,
ii) in a second scrubbing step (WS-II), the prescrubbed crude mixture comprising dinitrotoluene is extracted with a scrubbing acid II in a scrub comprising at least one, preferably at least 2, extraction stage(s), where the scrubbing acid discharged from the first extraction stage (WS-II-1) of the second scrubbing step (WS-II) has a pH of less than or equal to 4 and a content of nitric acid of not more than 2000 ppm, a content of nitrogen oxides (calculated as HNO₂) of not more than 50 ppm and a content of hydrocyanic acid of not more than 120 ppm, giving a dinitrotoluene-comprising mixture which is essentially free of nitric acid, sulfuric acid, nitrogen oxides and hydrocyanic acid and has a content of not more than 300 ppm of nitric acid and nitrogen oxides, not more than 3 ppm of sulfate and not more than 50 ppm of hydrocyanic acid.

2. The process according to claim 1, wherein the first scrubbing step (WS-I) comprises from 2 to 4 extraction stages and is carried out in countercurrent.

3. The process according to claim 1 or 2, wherein the second scrubbing step (WS-II) comprises from 2 to 6 extraction stages and is carried out in countercurrent.

4. The process according to any of claims 1 to 3, wherein the scrubbing acid discharged from the first extracton stage (WS-II-1) of the second scrubbing step (WS-II) has a pH of from 0 to 3.

5. The process according to any of claims 1 to 4, wherein the scrubbing acid discharged from the first extraction stage (WS-II-1) of the second scrubbing step (WS-II) has a pH of from 0.5 to 2.

6. The process according to any of claims 1 to 5, wherein water or a scrubbing acid having a total acid content of from 0.2 to 1.5% by weight is fed to the last extraction stage (WS-I-n) of the first scrubbing step (WS-I).

7. The process according to any of claims 1 to 6, wherein water is fed to the last extraction stage (WS-II-n) of the second scrubbing step (WS-II).

8. The process according to any of claims 1 to 7,
wherein
I) in the first extraction stage (WS-I-1) of the first scrubbing step (WS-I), the crude mixture comprising dinitrotoluene, nitric acid, nitrogen oxides and sulfuric acid is fed together with circulating scrubbing acid (WSR-I-1) from a first phase separation apparatus (S-I-1) belonging to this first extraction stage and excess scrubbing acid (WSR-I-2) from the subsequent extraction stage (WS-I-2) into a first mixer (M-I-1), the scrubbing emulsion formed in the mixer is separated in the first phase separation apparatus (S-I-1) into the scrubbing acid (WSR-I-1) and crude mixture (DNT-I-1) which has been scrubbed once, and
II) the crude mixture (DNT-I-1) which has been scrubbed once is, in the subsequent extraction stage (WS-I-2) of the first scrubbing step (WS-I), fed together with the circulated scrubbing acid (WSR-I-2) from a second phase separation apparatus (S-I-2) belonging to this extraction stage and, when the first scrubbing step (WS-I) comprises more than 2 extraction stages to (WS-I-n), with excess scrubbing acid (WSR-I-3) (WSR-I-n) from the subsequent extraction stage (WS-I-3) to (WS-I-n) or, when the first scrubbing step comprises precisely 2 extraction stages, with freshly introduced water or scrubbing acid (W-I-1) into a second mixer (M-I-2) belonging to this extraction stage.

9. The process according to any of claims 1 to 8,
wherein
I) in the first extraction stage (WS-II-1) of the second scrubbing step (WS-II), the prescrubbed crude mixture comprising dinitrotoluene is fed together with circulating scrubbing acid (WSR-II-1) from a first phase separation apparatus (S-II-1) belonging to this extraction stage and excess scrubbing acid (WSR-II-2) from the subsequent extraction stage (WS-II-2) into a first mixer (M-II-1) belonging to this extraction stage, the scrubbing emulsion formed in the first mixer is separated in the first phase separation apparatus (S-II-1) into the scrubbing acid (WSR-II-1) and crude mixture (DNT-II-1) which has been scrubbed once, and
II) the crude mixture (DNT-II-1) which has been scrubbed once is, in the subsequent extraction stage (WS-II-2) of the second scrubbing step (WS-II), fed together with the circulated scrubbing acid (WSR-II-2) from a second phase separation apparatus (S-II-2) belonging to this second extraction stage and, when the second scrubbing step (WS-II) comprises more than 2 extraction stages to (WS-II-m), with excess scrubbing acid (WSR-II-3) to (WSR-II-m) from the subsequent extraction stage (WS-II-3) to (WS-II-m) or, when the scrubbing step comprises precisely two extraction stages, with freshly introduced water (W-II-1) into a second mixer (M-II-2) belonging to this extraction stage of the second scrubbing step (WS-II).

10. The process according to any of claims 1-9, wherein the volume ratio of dinitrotoluene-comprising crude mixture and scrubbing acid in the extraction stages of the scrubbing steps (W-I) and (W-II) is from 1:4 to 10:1.

11. The process according to any of claims 1-10, wherein the volume ratio of dinitrotoluene-comprising crude mixture to freshly introduced water in the last extraction stage (W-II-m) of the second scrubbing step (W-II) is from 1:2 to 15:1.

12. The process according to any of claims 1 to 11, wherein a dinitrotoluene-comprising mixture which comprises a total of not more than 800 ppm of nitrocresols and nitrophenols, not more than 600 ppm of nitrobenzoic acids, not more than 300 ppm of nitric acid, not more than 50 ppm of hydrocyanic acid and not more than 3 ppm of sulfate is taken off from the last extraction stage (WS-II-m) of the second scrubbing step (WS-II).

13. The process according to any of claims 1 to 12, wherein the scrubbing acid discharged from the first extraction stage (WS-II-1) of the second scrubbing step (WS-II) has a total content of nitrocresols and nitrophenols of less than 5 ppm and a content of nitrobenzoic acids of less than 50 ppm.

14. The process according to claim 12 or 13, wherein the scrubbing acid discharged from the first extraction stage (WS-II-1) of the second scrubbing step (WS-II) is, after dinitrotoluene dissolved therein has been separated off by extraction and the extractant has been separated off and after neutralization, fed without further pretreatment to the biological treatment stage of a water treatment plant.

15. The process according to claim 14, wherein the scrubbing acid which has been separated off from the first extraction stage (WS-II-1) of the second scrubbing step (WS-II) is extracted with toluene and the extracted scrubbing acid is stripped with a stripping gas, giving a wastewater having a hydrocyanic acid content of less than 1 ppm.

## Revendications

1. Procédé de lavage d'un mélange brut formé lors de la nitration de toluène après la séparation de l'acide sulfonitrique, contenant du dinitrotoluène, de l'acide nitrique, des oxydes d'azote et de l'acide sulfurique, comprenant deux étapes de lavage (WS-I) et (WS-II), selon lequel
i) lors d'une première étape de lavage (WS-I), le mélange brut est extrait lors d'un lavage comprenant au moins une étape d'extraction avec un acide de lavage I contenant de l'acide nitrique, des oxydes d'azote et de l'acide sulfurique, l'acide de lavage déchargé de la première étape d'extraction (WS-I-1) de la première étape de lavage (WS-I) présentant une teneur totale en acide de 10 à 40 % en poids et une teneur de 80 à 350 ppm en acide cyanhydrique, un mélange brut prélavé étant obtenu,
ii) lors d'une deuxième étape de lavage (WS-II), le mélange brut prélavé contenant du dinitrotoluène est extrait lors d'un lavage comprenant au moins une, de préférence au moins 2 étapes d'extraction avec un acide de lavage II, l'acide de lavage déchargé de la première étape d'extraction (WS-II-1) de la deuxième étape de lavage (WS-II) présentant un pH inférieur ou égal à 4 et une teneur en sulfates d'au plus 100 ppm, une teneur en acide nitrique d'au plus 2 000 ppm, une teneur en oxydes d'azote (calculée en tant qu'HNO₂) d'au plus 50 ppm et une teneur en acide cyanhydrique d'au plus 120 ppm, un mélange essentiellement exempt d'acide nitrique, d'acide sulfurique, d'oxydes d'azote et d'acide cyanhydrique, contenant du dinitrotoluène, ayant une teneur d'au plus 300 ppm en acide nitrique et en oxydes d'azote, d'au plus 3 ppm en sulfates et d'au plus 50 ppm en acide cyanhydrique étant obtenu.

2. Procédé selon la revendication 1, **caractérisé en ce que** la première étape de lavage (WS-I) comprend 2 à 4 étapes d'extraction et est réalisée à contre-courant.

3. Procédé selon la revendication 1 ou 2, **caractérisé en ce que** la deuxième étape de lavage (WS-II) comprend 2 à 6 étapes d'extraction et est réalisée à contre-courant.

4. Procédé selon l'une quelconque des revendications 1 à 3, **caractérisé en ce que** l'acide de lavage déchargé de la première étape d'extraction (WS-II-1) de la deuxième étape de lavage (WS-II) présente un pH de 0 à 3.

5. Procédé selon l'une quelconque des revendications 1 à 4, **caractérisé en ce que** l'acide de lavage déchargé de la première étape d'extraction (WS-II-1) de la deuxième étape de lavage (WS-II) présente un pH de 0,5 à 2.

6. Procédé selon l'une quelconque des revendications 1 à 5, **caractérisé en ce que** de l'eau ou un acide de lavage ayant une teneur totale en acide de 0,2 à 1,5 % en poids est introduit dans la dernière étape d'extraction (WS-I-n) de la première étape de lavage (WS-I).

7. Procédé selon l'une quelconque des revendications 1 à 6, **caractérisé en ce que** de l'eau est introduite dans la dernière étape d'extraction (WS-II-n) de la deuxième étape de lavage (WS-II).

8. Procédé selon l'une quelconque des revendications 1 à 7, **caractérisé en ce que**
I) lors de la première étape d'extraction (WS-I-1) de la première étape de lavage (WS-I), le mélange brut contenant du dinitrotoluène, de l'acide nitrique, des oxydes d'azote et de l'acide sulfurique est introduit dans un premier mélangeur (M-I-1) conjointement avec un acide de lavage (WSR-I-1) mis en circulation issu d'un premier appareil de séparation de phases (S-I-1) appartenant à cette première étape d'extraction et un acide de lavage en excès (WSR-I-2) issu de l'étape d'extraction suivante (WS-I-2), l'émulsion de lavage formée dans le mélangeur est séparée dans le premier appareil de séparation de phases (S-I-1) en l'acide de lavage (WSR-I-1) et le mélange brut lavé une fois (DNT-I-1), et
II) le mélange brut lavé une fois (DNT-I-1) est introduit dans l'étape d'extraction suivante (WS-I-2) de la première étape de lavage (WS-I) dans un deuxième mélangeur (M-I-2) appartenant à cette étape d'extraction, conjointement avec l'acide de lavage (WSR-I-2) mis en circulation issu d'un deuxième appareil de séparation de phases (S-I-2) appartenant à cette étape d'extraction et, lorsque la première étape de lavage (WS-I) comprend plus de 2 étapes d'extraction à (WS-I-n), avec un acide de lavage en excès (WSR-I-3) à (WSR-I-n) issu de l'étape d'extraction suivante (WS-I-3) à (WS-I-n) ou, lorsque la première étape de lavage comprend exactement 2 étapes d'extraction, avec de l'eau fraîche ou l'acide de lavage (W-I-1).

9. Procédé selon l'une quelconque des revendications 1 à 8, **caractérisé en ce que**
I) lors de la première étape d'extraction (WS-II-1) de la deuxième étape de lavage (WS-II), le mélange brut prélavé contenant du dinitrotoluène est introduit dans un premier mélangeur (M-II-1) appartenant à cette étape d'extraction conjointement avec un acide de lavage (WSR-II-1) mis en circulation issu d'un premier appareil de séparation de phases (S-II-1) appartenant à cette étape d'extraction et un acide de lavage en excès (WSR-II-2) issu de l'étape d'extraction suivante (WS-II-2), l'émulsion de lavage formée dans le premier mélangeur est séparée dans le premier appareil de séparation de phases (S-II-1) en l'acide de lavage (WSR-II-1) et le mélange brut lavé une fois (DNT-II-1), et
II) le mélange brut lavé une fois (DNT-II-1) est introduit dans l'étape d'extraction suivante (WS-II-2) de la deuxième étape de lavage (WS-II) dans un deuxième mélangeur (M-II-2) appartenant à cette étape d'extraction, conjointement avec l'acide de lavage (WSR-II-2) mis en circulation issu d'un deuxième appareil de séparation de phases (S-II-2) appartenant à cette deuxième étape d'extraction et, lorsque la deuxième étape de lavage (WS-II) comprend plus de 2 étapes d'extraction à (WS-II-m), avec un acide de lavage en excès (WSR-II-3) à (WSR-II-m) issu de l'étape d'extraction suivante (WS-II-3) à (WS-II-m) ou, lorsque la première étape de lavage comprend exactement 2 étapes d'extraction, avec de l'eau fraîche (W-II-1).

10. Procédé selon l'une quelconque des revendications 1 à 9, **caractérisé en ce que** le rapport en volume entre le mélange brut contenant du dinitrotoluène et l'acide de lavage dans les étapes d'extraction des étapes de lavage (W-I) et (W-II) est de 1:4 à 10:1.

11. Procédé selon l'une quelconque des revendications 1 à 10, **caractérisé en ce que** le rapport en volume entre le mélange brut contenant du dinitrotoluène et l'eau fraîche dans la dernière étape d'extraction (W-II-m) de la deuxième étape de lavage (W-II) est de 1:2 à 15:1.

12. Procédé selon l'une quelconque des revendications 1 à 11, **caractérisé en ce qu'**un mélange contenant du dinitrotoluène est soutiré de la dernière étape d'extraction (WS-II-m) de la deuxième étape de lavage (WS-II), qui comprend au total au plus 800 ppm de nitrocrésols et nitrophénols, au plus 600 ppm d'acides nitrobenzoiques, au plus 300 ppm d'acide nitrique, au plus 50 ppm d'acide cyanhydrique et au plus 3 ppm de sulfates.

13. Procédé selon l'une quelconque des revendications 1 à 12, **caractérisé en ce que** l'acide de lavage déchargé de la première étape d'extraction (WS-II-1) de la deuxième étape de lavage (WS-II) présente une teneur en nitrocrésols et nitrophénols au total inférieure à 5 ppm et une teneur en acides nitrobenzoïques inférieure à 50 ppm.

14. Procédé selon la revendication 12 ou 13, **caractérisé en ce que** l'acide de lavage déchargé de la première étape d'extraction (WS-II-1) de la deuxième étape de lavage (WS-II) est introduit après la séparation du dinitrotoluène dissous dans celui-ci par extraction et la séparation de l'agent d'extraction et après une neutralisation sans prétraitement supplémentaire dans l'étape de traitement biologique d'une station d'épuration.

15. Procédé selon la revendication 14, **caractérisé en ce que** l'acide de lavage séparé de la première étape d'extraction (WS-II-1) de la deuxième étape de lavage (WS-II) est extrait avec du toluène et l'acide de lavage extrait est extrait avec un gaz d'extraction, une eau résiduaire ayant une teneur en acide cyanhydrique inférieure à 1 ppm étant obtenue.
